# EUROPEAN PATENT SPECIFICATION

(11) **EP 0 523 523 B1**
(45) Date of publication and mention of the grant of the patent: **24.05.1995**
(21) Application number: 92111552.3
(22) Date of filing: 08.07.1992
(51) Int. Cl.: A23K 1/17, A61K 31/505, A61K 31/35

(54) **Anticoccidial compositions**
Zusammensetzungen gegen Coccidiosis
Compositions contre la coccidiose

(30) Priority: 17.07.1991 US 731683
(43) Date of publication of application: 20.01.1993
(73) Proprietor: F. HOFFMANN-LA ROCHE AG, 4002 Basel (CH)
(72) Inventor: Schildknecht, Eugene George, Hackettstown, N.J. 07840 (US); Untawale, Govind Gajanan, Wayne, N.J. 07470 (US)
(74) Representative: Cottong, Norbert A.

(56) References cited:
- EP-A- 0 268 135
- US-A- 4 839 382

## Description

The invention relates to the field of compositions useful for the prevention and treatment of coccidiosis in animals, methods of using such compositions and animal comestibles containing such compositions.

Coccidiosis is a disease caused by microscopic protozoal parasites called coccidia, belonging to the genus Eimeria. The infection in the host animals is initiated by the ingestion, usually along with food, water, and/or fecal material, of Eimeria organisms in the sporulated oocyst stage. When the ingested oocysts enter the intestine, the infectious stage of the Eimeria soon develops from the oocysts and causes extensive damage to the inner walls of the intestine and the cecum or "intestinal pouch." Cecal coccidiosis in chickens, for example, is caused primarily by the organism E. tenella and results in the destruction of the cecal linings of the host.

A number of coccidiostatic agents are presently available for the prevention and/or treatment of coccidiosis. Still, many of these agents have certain shortcomings. Animals treated with some of the known coccidiostats sometimes show reduced feed efficiency and lower weight gains than normal. Moreover, the development of resistance to the more commonly used agents is becoming an increasingly significant problem, one which is becoming a limiting factor in successfully combating coccidiosis. Still other agents have very narrow safety and efficacy ranges with resulting toxicity risks for treated animals, as well as for other farm animals and man by virtue of incidental or accidental exposure or ingestion.

A naphthoquinone antibiotic produced from Streptomyces roseofulvus, frenolicin B, has been shown to have some anticoccidial activity against E. tenella in chickens (Omura, et al., J. Antibiotics, Vol. 38, No. 10, pp. 1447-8 (1985)). Frenolicin B has recently been shown, e.g. in U.S. Patent Specification No. 4,839,382, to be synergistic in combination with ionophoric anticoccidial antibiotics against which Eimeria field isolates had developed resistance due to the chronic usage of these agents to combat coccidiosis.

It has now been discovered that compositions that contain a combination of frenolicin B and the anticoccidial agent halofuginone hydrobromide result in significantly potentiated activity against coccidiosis caused by Eimeria that have developed resistance to this anticoccidial agent. The activity of the present compositions is greater than would be expected from a simple additive effect of the two components, i.e. is synergistic.

Accordingly, the present invention is directed to anticoccidial compositions, i.e compositions which are useful for combating, i.e preventing and treating, coccidiosis in animals, comprising a mixture of frenolicin B and halofuginone hydrobromide, wherein these ingredients are present in amounts which in combination are synergistically effective in combating at least one coccidiosis-causing strain of Eimeria.

These compositions are especially useful by virtue of their potentiated activity against strains of Eimeria which have become resistant to the aforementioned chemical anticoccidial which has been approved for and used in the treatment of coccidiosis, especially in poultry.

The compositions of the present invention, when administered to animals such as domestic animals and animals raised commercially for food, such as poultry, cattle, sheep and pigs, are effective in the prophylaxis and treatment of coccidiosis. The frenolicin B synergistically interacts with the halofuginone hydrobromide component of the present compositions resulting in greater activity against resistant coccidiosis. Another advantage of the present invention is that the use of the frenolicin B makes possible the use of reduced amounts of the active ingredients with resulting reduction in the risk of toxicity and the side effects typically associated therewith, such as adverse influence on feeding, water intake and nutrient absorption.

The production and isolation from Streptomyces roseofulvus of the frenolicin B utilized in the present invention is disclosed in the article by Iwai et al. in J. Antibiotics, Vol. 31, No. 10, pp. 959-965 (1978).

Halofuginone hydrobromide, i.e. (+)-trans-7-bromo-6-chloro-3-[3-(3-hydroxy-2-piperidyl)acetonyl]-4-(3H)-quinazolinone hydrobromide, is a known and commercially available anticoccidial.

The individual components of the compositions of the present invention are employed in relative amounts which are synergistic in combatting coccidiosis-producing microorganisms and in particular against those strains of Eimeria which have developed resistance to the halofuginone anticoccidial due to exposure over time to the latter. A preferred weight ratio range of frenolicin B to halofuginone hydrobromide, i.e. one which results in particularly good synergistic effects, is from about 75:1 to about 11:1.

The present invention further embraces improved anticoccidial animal comestible and drinking water compositions. Such anticoccidial compositions comprise an animal feed or drinking water, as appropriate, in admixture with (or containing) an anticoccidial composition as defined hereinabove, wherein the halofuginone hydrobromide is present in an amount from about 1 to about 3 ppm by weight of the animal feed or drinking water component, and the frenolicin B is present in an amount which combined with the amount of halofuginone hydrobromide is synergistically effective in combating at least one coccidiosis-causing strain of Eimeria. These anticoccidial animal comestible or drinking water compositions may be prepared by mixing the active ingredients with suitable carrier or diluent material generally used as animal feed, or with drinking water, as appropriate.

The concentration of the active ingredients in the anticoccidial animal comestible or drinking water compositions of this invention can be adjusted to meet particular needs and may vary over a wide range. The limiting criteria of the concentration are that the minimum concentration is such that a sufficient amount of the active ingredients is provided to effect the desired control of coccidiosis and the maximum concentration is such that the amount of composition ingested does not result in any untoward or undesirable side effects. Because of the potentiating interaction of the active ingredients of the present invention, the amounts of each component required may usefully be less than that which would ordinarily be considered the usual recommended dosing level for the individual components if used separately as a coccidiostat. The amount of the halofuginone hydrobromide necessary for successfully combating coccidiosis may therefore be beneficially reduced. Thus, the halofuginone hydrobromide component is typically present in an amount of from about 25 to about 90%, and preferably 33% to 75%, of the normal recommended dosing level for that anticoccidial agent. For example, the approved recommended dosing level for halofuginone hydrobromide for the prevention of coccidiosis in chickens would be about 3 ppm The compositions in accordance with the present invention can accordingly contain the halofuginone hydrobromide in amounts of from as little as about 1 ppm depending on the respective dose of frenolicin B, that is from about 33 to about 75 ppm, based on the weight of the animal feed or drinking water component.

The easiest way to administer the anticoccidial compositions of the present invention is by providing the animals to be treated with an anticoccidial animal comestible or drinking water composition of the present invention. However, the anticoccidial compositions can be usefully administered in other ways. For example, they can be incorporated into tablets, drenches, boluses, or capsules, and dosed to the animals. Formulations of the antibiotic compounds in such dosage forms can be accomplished by means of methods well-known in the veterinary pharmaceutical art. Of course, whatever the method or methods of administration, the two components of the anticoccidial compositions of the present invention can be administered independently so long as the synergistically effective combination is ultimately dosed to the animal. All these methods of administration represent further aspects of the present invention.

As mentioned hereinbefore, the most practical way to treat animals with the anticoccidial compositions is by providing the animals with the above-defined anticoccidial comestible or drinking water compositions. Any type of feed may be medicated with the anticoccidial compositions, including common dry feeds, liquid feeds and pelleted feeds.

The methods of formulating anticoccidials into animal feeds are well-known. It is usual to make a concentrated anticoccidial premix as a raw material for medicated, in the present case anticoccidial, feeds. For example, typical anticoccidial premixes may contain from about 2.0 to about 150 grams of anticoccidial per pound of premix (approx. 4.4 g/kg - 331 g/kg). The wide range results from the wide range of concentration of anticoccidial which may be desired in the final feed. The animal feed additive premix embraced by the present invention comprises about 4.4 g/kg to about 331 g/kg of an anticoccidial composition, said composition consisting of a mixture of frenolicin B and halofuginone hydrobromide present in amounts which in combination are synergistically effective in combating at least one coccidiosis-causing strain of Eimeria. These premixes may be either liquid or dry formulations, and contain, apart from the aforementioned active ingredients, conventional edible carrier material suitable for the animals concerned. As in the case of the anticoccidial compositions, animal comestible compositions and animal drinking water compositions of the present invention the preferred weight ratio range of frenolicin B to halofuginone hydrobromide is from about 75:1 to about 11:1.

The formulation of animal feeds containing the proper amounts of anticoccidial composition for useful treatment can be effected in conventional manner. It is necessary only to calculate the amount of composition which it is desired to administer to the animals in question, taking into account the amount of feed per day which the animals eat and the concentration of anticoccidial composition (mixture of the active ingredients) as such or in the premix to be used, and calculate the appropriate concentration of anticoccidial composition or of premix in the feed and thus the dilution factor.

The known methods of formulating, mixing and processing feeds which are normally used in the animal feed arts are entirely appropriate for manufacturing feeds containing the anticoccidial compositions of the present invention. For example, separately formulated premixes for each of the two components could be added to a given feed lot to provide an anticoccidial animal comestible composition of the present invention. Analogous considerations apply for the formulation of the anticoccidial drinking water compositions of the present invention.

The compositions of the present invention are typically effective in combating coccidiosis when administered to animals in feed mixes containing the compositions (mixture of the two active ingredients) in a concentration of from about 35 ppm to about 75 ppm by weight.

The following examples are illustrative of the invention.

### Example 1

### Anticoccidial Activity against Halofuginone Hydrobromide Resistant Field Strain of E. tenella

### Experimental Procedure

Two-week old Arbor Acres, Peterson Cross broiler chickens, obtained from a commercial hatchery and kept in wire-floored, electrically heated, battery brooders, were used in all studies. Male birds, selected according to weight, were used in each group. The chickens were medicated two days before infection and maintained on the medicated feed until the termination of the trial for a total medication period of nine days. There were two replicates of eight birds each per treatment.

### Eimeria Field Isolates

The E. tenella field isolate was recovered from a commercial broiler operation in North Carolina. The intestinal samples were homogenized with a Waring Blender, suspended in 2.5% potassium dichromate solution, sporulated at room temperature, and inoculated into young susceptible, coccidia-free chicks for further oocyst collection and species identification.

At appropriate post-infection times, 5 to 7 days, the birds were sacrificed, autopsied, and diagnosis of Eimeria species made, based on location site, and oocysts measurement. Oocysts were collected from the intestines of the passaged birds and sporulated.

In evaluating the efficacy of the frenolicin B combinations, 3.0 x 10⁵ sporulated oocysts were administered per bird. The sporulated oocysts, properly agitated and suspended in sterile distilled water, were inoculated in volumes of 1.0 ml directly into the chick crop by means of a blunt needle attached to a calibrated syringe.

### Criteria for Evaluating Anticoccidial Efficacy

At termination of the trials, the surviving birds were sacrificed, necropsied and scored for gross lesions. All birds that died during the experiments were also necropsied; proper diagnosis was made, and their gross lesions scored and recorded. The intestinal lesions were scored as 0 = normal, 1 = slight, 2 = moderate, 3 = severe and 4 = dead. The readings obtained were summarized as average degree of infection (ADI).

In addition, bird group weight, feed intake, feed conversion and mortality were recorded.

### Results

The results are set forth in Table 1 below:

**Table 1**

| Anticoccidial Activity of Frenolicin B in Combination with Halofuginone HBr against Eimeria tenella | | | | | |
|---|---|---|---|---|---|
| Treatment | Level (ppm) | Weight Gain. % | Feed Conversion. | Lesion Score | % Mortality |
| UUC¹ | 0 | 100 | 1.62 | 0.0 | 0 |
| IUC² | 0 | 67 | 2.07 | 3.1 | 25 |
| Frenolicin B | 60 | 100 | 1.58 | 1.1 | 0 |
| Halofuginone HBr | 3 | 68 | 2.25 | 3.3 | 37.5 |
| Halofuginone HBr + Frenolicin B | 3 + 60 | 96 | 1.60 | 0.6 | 0 |
| Halofuginone HBr + Frenolicin B | 3 + 30 | 98 | 1.68 | 1.6 | 0 |
| Halofuginone HBr + Frenolicin B | 1.5 + 60 | 98 | 1.66 | 0.8 | 0 |
| Halofuginone HBr + Frenolicin B | 1.5 + 30 | 102 | 1.63 | 1.7 | 0 |

| | | | | | |
|---|---|---|---|---|---|
| ¹UUC = uninfected unmedicated chickens | | | | | |
| ²IUC = infected unmedicated chickens | | | | | |

### Example 2

A typical animal feed additive premix contains 22.7 g frenolicin B/lb premix (approx. 50.05 g/kg) and 2.72 g halofuginone hydrobromide/lb premix (approx. 6.00 g/kg), the rest being made up of rice bran, calcium carbonate and optionally other edible carrier materials(s). The nature and relative contents of the edible carrier materials can be varied at will as appropriate for the type of animals for which the anticoccidial comestible composition, made up from the premix, is ultimately intended.

Such and other animal feed additive premixes can be added, e.g. at the rate of 1 lb (0.454 kg) ton, to an animal feed, e.g. of the following compositions, to produce typical anticoccidial animal comestible compositions for very young ("starter"), growing ("grower") and fully grown ("finisher") poultry (chickens, hens):

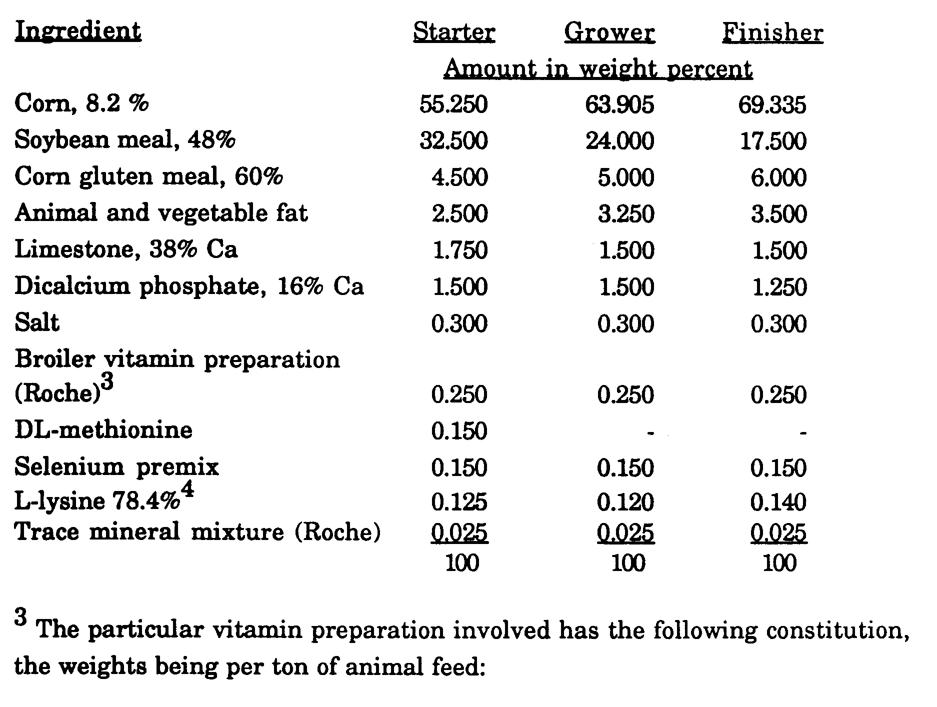

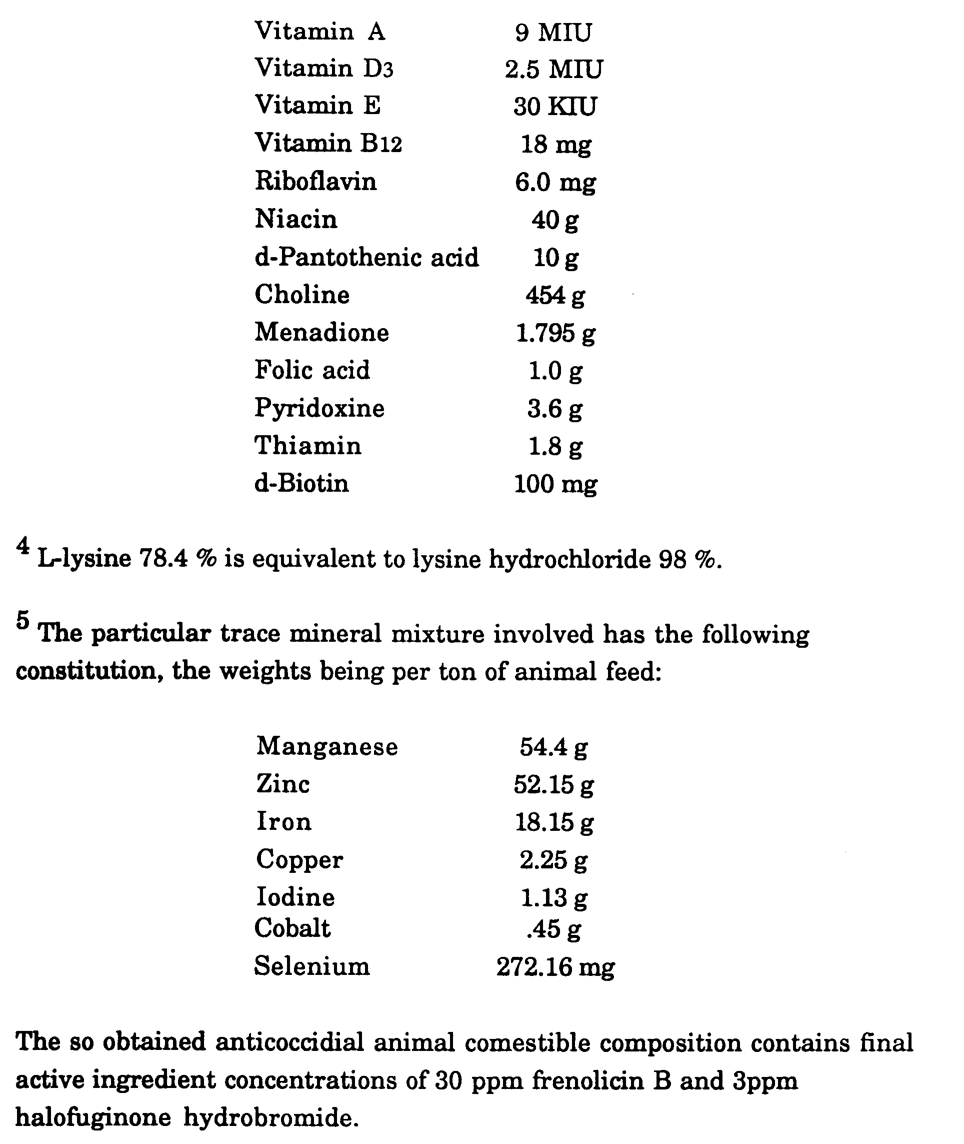

## Claims (Claims for the following Contracting State(s): AT, BE, CH, DE, DK, FR, GB, IT, LI, NL)

1. A composition for combating coccidiosis in animals comprising a mixture of frenolicin B and halofuginone hydrobromide, wherein these ingredients are present in amounts which in combination are synergistically effective in combating at least one coccidiosis-causing strain of Eimeria.

2. A composition according to claim 1, wherein the weight ratio of frenolicin B to halofuginone hydrobromide is in the range from about 75:1 to about 11:1.

3. An anticoccidial animal comestible composition comprising an animal feed in admixture with a composition for combating coccidiosis in animals, said latter composition comprising a mixture of frenolicin B and halofuginone hydrobromide, wherein the halofuginone hydrobromide is present in an amount from about 1 to about 3 ppm by weight of the animal feed component and the frenolicin B is present in an amount which combined with the amount of halofuginone hydrobromide is synergistically effective in combating at least one coccidiosis-causing strain of Eimeria.

4. A composition according to claim 3, wherein the amount of frenolicin B is from about 33 to about 75 ppm by weight of the animal feed.

5. An anticoccidial animal drinking water composition comprising drinking water containing a composition for combating coccidiosis in animals, said latter composition comprising a mixture of frenolicin B and halofuginone hydrobromide, wherein the halofuginone hydrobromide is present in an amount from about 1 to about 3 ppm by weight of the drinking water component and the frenolicin B is present in an amount which combined with the amount of halofuginone hydrobromide is synergistically effective in combating at least one coccidiosis-causing strain of Eimeria.

6. An animal feed additive premix comprising about 4.4 g/kg to about 331 g/kg of an anticoccidial composition, said composition consisting of a mixture of frenolicin B and halofuginone hydrobromide present in amounts which in combination are synergistically effective in combating at least one coccidiosis-causing strain of Eimeria.

7. An animal feed additive premix according to claim 6, wherein the weight ratio of frenolicin B to halofuginone hydrobromide is in the range from about 75:1 to about 11:1.

## Claims (Claims for the following Contracting State(s): ES)

1. A process for producing a composition for combating coccidiosis in animals comprising admixing frenolicin B and halofuginone hydrobromide in amounts which in combination are synergistically effective in combating at least one coccidiosis-causing strain of Eimeria.

2. A process according to claim 1, wherein the weight ratio of frenolicin B to halofuginone hydrobromide is in the range from about 75:1 to about 11:1.

3. A process for producing an anticoccidial animal comestible composition comprising admixing an animal feed with a composition for combating coccidiosis in animals, said latter composition comprising a mixture of frenolicin B and halofuginone hydrobromide, wherein the halofuginone hydrobromide is present in an amount from about 1 to about 3 ppm by weight of the animal feed component and the frenolicin B is present in an amount which combined with the amount of halofuginone hydrobromide is synergistically effective in combating at least one coccidiosis-causing strain of Eimeria.

4. A process according to claim 3, wherein the amount of frenolicin B is from about 33 to about 75 ppm by weight of the animal feed.

5. A process for producing an anticoccidial animal drinking water composition comprising admixing drinking water with a composition for combating coccidiosis in animals, said latter composition comprising a mixture of frenolicin B and halofuginone hydrobromide, wherein the halofuginone hydrobromide is present in an amount from about 1 to about 3 ppm by weight of the drinking water component and the frenolicin B is present in an amount which combined with the amount of halofuginone hydrobromide is synergistically effective in combating at least one coccidiosis-causing strain of Eimeria.

6. A process for producing an animal feed additive premix comprising admixing about 4.4 g/kg to about 331 g/kg of an anticoccidial composition,
said composition consisting of a mixture of frenolicin B and halofuginone hydrobromide present in amounts which in combination are synergistically effective in combating at least one coccidiosis-causing strain of Eimeria.

7. A process according to claim 6, wherein the weight ratio of frenolicin B to halofuginone hydrobromide is in the range from about 75:1 to about 11:1.

## Patentansprüche (Patentansprüche für folgende(n) Vertragsstaat(en): AT, BE, CH, DE, DK, FR, GB, IT, LI, NL)

1. Zusammensetzung zur Bekämpfung von Kokzidiose in Tieren enthaltend ein Gemisch von Frenolicin B und Halofuginon-Hydrobromid, wobei diese Bestandteile in Mengen vorhanden sind, die in Kombination synergistisch wirksam sind zur Bekämpfung von mindestens einem Kokzidiose hervorrufenden Stamm von Eimeria.

2. Zusammensetzung nach Anspruch 1, worin das Gewichtsverhältnis Frenolicin B zu Halofuginon-Hydrobromid im Bereich von etwa 75:1 bis etwa 11:1 liegt.

3. Antikokzidiose-Tierfuttermittel enthaltend ein Tierfutter in Gemisch mit einer Zusammensetzung zur Bekämpfung von Kokzidiose in Tieren, wobei die besagte Zusammensetzung ein Gemisch von Frenolicin B und Halofuginon-Hydrobromid enthält, in welchem das Halofuginon-Hydrobromid in einer Menge von etwa 1 bis etwa 3 ppm bezogen auf das Gewicht der Tierfutterkomponente vorliegt und das Frenolicin B in einer Menge vorhanden ist, die in Kombination mit der Menge Halofuginon-Hydrobromid synergistisch wirksam ist zur Bekämpfung von mindestens einem Kokzidiose hervorrufenden Stamm von Eimeria.

4. Mittel nach Anspruch 3, worin die Menge Frenolicin B etwa 33 bis etwa 75 ppm bezogen auf das Gewicht des Tierfutters beträgt.

5. Antikokzidiose-Trinkwassermittel für Tiere enthaltend Trinkwasser, das eine Zusammensetzung zur Bekämpfung von Kokzidiose in Tieren enthält, wobei die besagte Zusammensetzung ein Gemisch von Frenolicin B und Halofuginon-Hydrobromid enthält, in welchem das Halofuginon-Hydrobromid in einer Menge von etwa 1 bis etwa 3 ppm bezogen auf das Gewicht der Trinkwasserkomponente vorliegt und das Frenolicin B in einer Menge vorhanden ist, die in Kombination mit der Menge Halofuginon-Hydrobromid synergistisch wirksam ist zur Bekämpfung von mindestens einem Kokzidiose hervorrufenden Stamm von Eimeria.

6. Tierfutterzusatz-Prämix enthaltend etwa 4,4 g/kg bis etwa 331 g/kg eines Antikokzidiosemittels, wobei das besagte Mittel aus einem Gemisch von Frenolicin B und Halofuginon-Hydrobromid besteht, und zwar in Mengen, die in Kombination synergistisch wirksam sind zur Bekämpfung von mindestens einem Kokzidiose hervorrufenden Stamm von Eimeria.

7. Tierfutterzusatz-Prämix nach Anspruch 6, worin das Gewichtsverhältnis Frenolicin B zu Halofuginon-Hydrobromid im Bereich von etwa 75:1 bis etwa 11:1 liegt.

## Patentansprüche (Patentansprüche für folgende(n) Vertragsstaat(en): ES)

1. Verfahren zur Herstellung einer Zusammensetzung zur Bekämpfung von Kokzidiose in Tieren, wonach man Frenolicin B und Halofuginon-Hydrobromid zusammenmischt in Mengen, die in Kombination synergistisch wirksam sind zur Bekämpfung von mindestens einem Kokzidiose hervorrufenden Stamm von Eimeria.

2. Verfahren nach Anspruch 1, worin das Gewichtsverhältnis Frenolicin B zu Halofuginon-Hydrobromid im Bereich von etwa 75:1 bis etwa 11:1 liegt.

3. Verfahren zur Herstellung eines Antikokzidiose-Tierfuttermittels. wonach man ein Tierfutter mit einer Zusammensetzung zur Bekämpfung von Kokzidiose in Tieren zusammenmischt, wobei die besagte Zusammensetzung ein Gemisch von Frenolicin B und Halofuginon-Hydrobromid enthält, in welchem das Halofuginon-Hydrobromid in einer Menge von etwa 1 bis etwa 3 ppm bezogen auf das Gewicht der Tierfutterkomponente vorliegt und das Frenolicin B in einer Menge vorhanden ist, die in Kombination mit der Menge Halofuginon-Hydrobromid synergistisch wirksam ist zur Bekämpfung von mindestens einem Kokzidiose hervorrufenden Stamm von Eimeria.

4. Verfahren nach Anspruch 3, worin die Menge Frenolicin B etwa 33 bis etwa 75 ppm bezogen auf das Gewicht des Tierfutters beträgt.

5. Verfahren zur Herstellung eines Antikokzidiose-Trinkwassermittels, wonach man Trinkwasser mit einer Zusammensetzung zur Bekämpfung von Kokzidiose in Tieren zusammenmischt, wobei die besagte Zusammensetzung ein Gemisch von Frenolicin B und Halofuginon-Hydrobromid enthält, in welchem das Halofuginon-Hydrobromid in einer Menge von etwa 1 bis etwa 3 ppm bezogen auf das Gewicht der Trinkwasserkomponente vorliegt und das Frenolicin B in einer Menge vorhanden ist, die in Kombination mit der Menge Halofuginon-Hydrobromid synergistisch wirksam ist zur Bekämpfung von mindestens einem Kokzidiose hervorrufenden Stamm von Eimeria.

6. Verfahren zur Herstellung eines Tierfutterzusatz-Prämix, wonach man etwa 4,4 g/kg bis etwa 331 g/kg einer Antikokzidiose-Zusammensetzung (sic) zusammenmischt, wobei die besagte Zusammensetzung aus einem Gemisch von Frenolicin B und Halofuginon-Hydrobromid besteht, und zwar in Mengen, die in Kombination synergistisch wirksam sind zur Bekämpfung von mindestens einem Kokzidiose hervorrufenden Stamm von Eimeria.

7. Verfahren nach Anspruch 6, worin das Gewichtsverhältnis Frenolicin B zu Halofuginon-Hydrobromid im Bereich von etwa 75:1 bis etwa 11:1 liegt.

## Revendications (Revendications pour l'(les) Etat(s) contractant(s) suivant(s): AT, BE, CH, DE, DK, FR, GB, IT, LI, NL)

1. Composition destinée à combattre la coccidiose chez des animaux, comprenant un mélange de frénolicine B et d'halofuginone bromhydrate, dans laquelle ces composants sont présents en quantités qui, en association, sont efficaces de façon synergique pour lutter contre au moins une souche d'*Eimeria* provoquant la coccidiose.

2. Composition selon la revendication 1, dans laquelle le rapport pondéral de la frénolicine B à l'halofuginone bromhydrate est dans la plage allant d'environ 75:1 à environ 11:1.

3. Composition anticoccidienne comestible pour les animaux, comprenant un aliment pour animaux en mélange avec une composition destinée à combattre la coccidiose chez des animaux, cette dernière composition comprenant un mélange de frénolicine B et d'halofuginone bromhydrate, dans lequel l'halofuginone bromhydrate est présente en une quantité allant d'environ 1 à environ 3 ppm en poids du composant aliment pour animaux, et la frénolicine B est présente en une quantité qui, associée à la quantité d'halofuginone bromhydrate, est efficace de façon synergique pour lutter contre au moins une souche d'*Eimeria* provoquant la coccidiose.

4. Composition selon la revendication 3, dans laquelle la quantité de frénolicine B va d'environ 33 à environ 75 ppm en poids de l'aliment pour animaux.

5. Composition anticoccidienne d'eau de boisson pour animaux, comprenant de l'eau de boisson contenant une composition destinée à combattre la coccidiose chez des animaux, cette dernière composition comprenant un mélange de frénolicine B et d'halofuginone bromhydrate, dans lequel l'halofuginone bromhydrate est présente en une quantité allant d'environ 1 à environ 3 ppm en poids du composant eau de boisson, et la frénolicine B est présente en une quantité qui, associée à la quantité d'halofuginone bromhydrate, est efficace de façon synergique pour lutter contre au moins une souche d'*Eimeria* provoquant la coccidiose.

6. Prémélange d'additifs à un aliment pour animaux, comprenant d'environ 4,4 g/kg à environ 331 g/kg d'une composition anticoccidienne, ladite composition consistant en un mélange de frénolicine B et d'halofuginone bromhydrate présentes en quantités qui, en association, sont efficaces de façon synergique pour lutter contre au moins une souche d'*Eimeria* provoquant la coccidiose.

7. Prémélange d'additifs à un aliment pour animaux selon la revendication 6, dans lequel le rapport pondéral de la frénolicine B à l'halofuginone bromhydrate est dans la plage allant d'environ 75:1 à environ 11:1.

## Revendications (Revendications pour l'(les) Etat(s) contractant(s) suivant(s): ES)

1. Procédé pour la préparation d'une composition destinée à combattre la coccidiose chez des animaux, comprenant le mélange de frénolicine B et d'halofuginone bromhydrate en quantités qui, en association, sont efficaces de façon synergique pour lutter contre au moins une souche d'*Eimeria* provoquant la coccidiose.

2. Procédé selon la revendication 1, dans lequel le rapport pondéral de la frénolicine B à l'halofuginone bromhydrate est dans la plage allant d'environ 75:1 à environ 11:1.

3. Procédé pour la préparation d'une composition anticoccidienne comestible pour les animaux, comprenant le mélange d'un aliment pour animaux avec une composition destinée à combattre la coccidiose chez des animaux, cette dernière composition comprenant un mélange de frénolicine B et d'halofuginone bromhydrate, dans lequel l'halofuginone bromhydrate est présente en une quantité allant d'environ 1 à environ 3 ppm en poids du composant aliment pour animaux, et la frénolicine B est présente en une quantité qui, associée à la quantité d'halofuginone bromhydrate, est efficace de façon synergique pour lutter contre au moins une souche d'*Eimeria* provoquant la coccidiose.

4. Procédé selon la revendication 3, dans lequel la quantité de frénolicine B va d'environ 33 à environ 75 ppm en poids de l'aliment pour animaux.

5. Procédé pour la préparation d'une composition anticoccidienne d'eau de boisson pour animaux, comprenant le mélange d'eau de boisson avec une composition destinée à combattre la coccidiose chez des animaux, cette dernière composition comprenant un mélange de frénolicine B et d'halofuginone bromhydrate, dans lequel l'halofuginone bromhydrate est présente en une quantité allant d'environ 1 à environ 3 ppm en poids du composant eau de boisson, et la frénolicine B est présente en une quantité qui, associée à la quantité d'halofuginone bromhydrate, est efficace de façon synergique pour lutter contre au moins une souche d'*Eimeria* provoquant la coccidiose.

6. Procédé pour la préparation d'un prémélange d'additifs à un aliment pour animaux, comprenant le mélange d'environ 4,4 g/kg avec environ 331 g/kg d'une composition anticoccidienne, ladite composition consistant en un mélange de frénolicine B et d'halofuginone bromhydrate présentes en quantités qui, en association, sont efficaces de façon synergique pour lutter contre au moins une souche d'*Eimeria* provoquant la coccidiose.

7. Procédé selon la revendication 6, dans lequel le rapport pondéral de la frénolicine B à l'halofuginone bromhydrate est dans la plage allant d'environ 75:1 à environ 11:1.
